# EUROPÄISCHE PATENTSCHRIFT

(11) **EP 2 686 304 B1**
(45) Veröffentlichungstag und Bekanntmachung des Hinweises auf die Patenterteilung: **18.05.2016**
(21) Anmeldenummer: 12707610.7
(22) Anmeldetag: 08.03.2012
(51) Int. Cl.: C07D 233/58, C07D 233/60

(54) **Verfahren zur Herstellung von ionischen Flüssigkeiten durch Anionenaustausch**
Process for preparing ionic liquids by anion exchange
Procédé pour la praparation de liquides ioniques par échange d'anions

(30) Priorität: 15.03.2011 EP 11158260
(43) Veröffentlichungstag der Anmeldung: 22.01.2014
(73) Patentinhaber: BASF SE, 67056 Ludwigshafen am Rhein (DE)
(72) Erfinder: KLEIN, Michael, 66879 Reichenbach-Steegen (DE); SIEMER, Michael, 68159 Mannheim (DE); FÜRST, Diana, 67071 Ludwigshafen (DE); FORSTER, Günter, 67063 Ludwigshafen (DE)
(74) Vertreter: BASF IP Association
(86) Internationale Anmeldenummer: PCT/EP2012/054043
(87) Internationale Veröffentlichungsnummer: WO 2012/123336

(56) Entgegenhaltungen:
- WO-A1-2009/059934
- WO-A2-2005/021484
- WO-A2-2009/027250
- WO-A2-2009/074535

## Beschreibung

Die vorliegende Anmeldung schließt durch Verweis die am 15. März 2011 einreichte vorläufige US-Anmeldung 61/452660 ein.

Die Erfindung betrifft ein Verfahren zur Herstellung von Salzen K⁺ X-, wobei es sich bei K⁺ um ein organisches Kation mit einem heterocyclischen Ringsystem und mindestens einem Stickstoffatom als Bestandteil des Ringsystems handelt und X⁻ ein Anion bezeichnet, durch Anionenaustausch, dadurch gekennzeichnet, dass
- von einem Salz K⁺ Y⁻ ausgegangen wird, wobei K⁺ die obige Bedeutung hat und Y⁻ für ein organisches Anion mit einer Carboxylat-, Sulfonat- oder Sulfatgruppe steht, das Anion Y⁻ insgesamt mindestens 4 C-Atome enthält und
- K⁺ Y⁻ mit einer Wasserstoffsäure HX umgesetzt wird, deren pKs - Wert kleiner ist als der pKs Wert der Wasserstoffsäure HY, und HX ausgewählt ist aus HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄.
- nach der Umsetzung das erhaltene Salz K⁺ X⁻ und die erhaltene Wasserstoffsäure HY in getrennten flüssigen Phasen vorliegen.

Bei Ionischen Flüssigkeiten handelt es sich um Salze mit einem Schmelzpunkt kleiner 100°C, insbesondere handelt es sich um Salze, welche bei Raumtemperatur flüssig sind.

Im Allgemeinen haben die ionischen Flüssigkeiten ein organisches Kation, genannt sei z.B. ein Imidazolium- Kation. Dieses organische Kation trägt wesentlich dazu bei, dass das Salz einen geringen Schmelzpunkt hat und bildet im Allgemeinen mit unterschiedlichsten Anionen eine ionische Flüssigkeit. Die Anionen haben naturgemäß starken Einfluss auf die anwendungstechnischen Eigenschaften von ionischen Flüssigkeiten, so dass für unterschiedliche technische Anwendungen entsprechend unterschiedliche Anionen geeignet oder bevorzugt sind.

Bei Verfahren zur Herstellung von ionischen Flüssigkeiten wird daher zunächst das organische Kation hergestellt, welches als Salz mit einem durch das Herstellverfahren bedingten Anions anfällt. Ausgehend von diesem Salz können unterschiedliche ionische Flüssigkeiten jeweils durch Anionenaustausch erhalten werden.

Ein derartiges Verfahren ist z.B. aus WO 2005/021484 bekannt. Danach ist zunächst die Herstellung eines Imidazols notwendig. Dieses Imidazol wird mit einem Dialkylcarbonat alkyliert, wodurch ein Salz aus Imidazolium - Kation und Carbonat-Anion erhalten wird. Bei Austausch des Carbonat-Anions gegen ein anderes gewünschtes Anion durch Zugabe einer Wasserstoffsäure zerfällt das Carbonat - Anion unter Bildung von CO₂ und Alkanol. Um ein Imidazoliumsalz mit einem gewünschten Anion herzustellen, sind daher drei Stufen notwendig (Herstellung Imidazol - Herstellung Imidazolium - Carbonat - Anionenaustausch).

WO 2009/059934 A1 offenbart ebenfalls ein Verfahren zur Herstellung ionischer Flüssigkeiten durch Anionenaustausch. Als Ausgangsprodukt werden beispielsweise Imidazolium-Acetat-Salze eingesetzt. Der lonenaustausch erfolgt in Anwesenheit von Ammoniak. Die Aufreinigung des Endprodukts erfolgt mittels Kurzwegdestillation. Das Produkt wird als Sumpfablauf erhalten.

In WO 91/14678 wird ein einstufiges Verfahren zur Herstellung von Imidazoliumsalzen aus einer α -Dicarbonylverbindung, einem Aldehyd, einem Amin und einer Säure beschrieben.

Eine verbesserte Ausgestaltung dieses Verfahrens ist aus WO 2009/074535 bekannt. Bei diesen Verfahren wird direkt ein salz mit Imidazolium - Kation in einer Stufe erhalten. Es besteht ie nach gewünschtem Anion weiterhin die Notwendigkeit eines Anionenaustausches, so dass das Verfahren dann insgesamt zweistufig ist. Bei einem Anionentausch können Salze z.B. in einen geeigneten, Hydroxydgruppen enthaltenden Anionentauscher gegeben werden. Dadurch wird der Anionentauscher mit den bisherigen Anionen beladen und die Salze enthalten nunmehr Hydroxydgruppen als Anionen. Durch Zusatz einer Wasserstoffsäure entsteht dann daraus Wasser und das Salz mit dem Anion der zugesetzten Wasserstoffsäure.

Aufgabe der vorliegenden Erfindung war ein möglichst einfaches und effektives Verfahren zur Herstellung von ionischen Flüssigkeiten, z.B. von Imidazoliumsalzen. Insbesondere sollen durch ein derartiges Verfahren ionische Flüssigkeiten mit unterschiedlichen Anionen leicht zugänglich sein.

Demgemäß wurde das eingangs definierte Verfahren gefunden.

Das Verfahren geht aus von einem Salz K⁺ Y⁻, wobei K⁺ für ein organisches Kation und Y⁻ für ein organisches Anion mit einer Carboxylat-, Sulfonat- oder Sulfatgruppe steht, wobei das Anion Y⁻ insgesamt mindestens 4 C-Atome enthält.

### Zum Kation K⁺

Die organische Kationen enthalten ein heterocyclisches Ringsystem , wobei mindestens ein, vorzugsweise ein bis drei Stickstoffatome, Bestandteil des Ringsystems sind.

In Betracht kommen monocyclische, bicyclische, aromatische oder nicht-aromatische Ringsysteme. Genannt seien z.B. bicyclische Systeme, wie sie in WO 2008/043837 beschrieben sind. Bei den bicyclischen Systemen der WO 2008/043837 handelt es sich um Diazabicyclo-Derivate, vorzugsweise aus einem 7- und einem 6 Ring, welche eine Amidiniumgruppe enthalten; genannt sei insbesondere das 1,8-Diazabicyclo(5.4.0)undec-7-enium- Kation.

Ganz besonders bevorzugte sind Kationen, die ein heterocyclisches Ringsystem mit ein oder zwei Stickstoffatomen als Bestandteil des Ringsystems enthalten.

Als derartige organische Kationen in Betracht kommen z.B. Pyridinium-Kationen, Pyridazinium-Kationen, Pyrimidinium-Kationen, Pyrazinium-Kationen, Imidazolium-Kationen, Pyrazolium-Kationen, Pyrazolinium-Kationen, Imidazolinium-Kationen, Thiazolium-Kationen, Triazolium-Kationen, Pyrrolidinium-Kationen und Imidazolidinium-Kationen. Diese Kationen sind z.B. in WO 2005/113702 aufgeführt. Soweit es für eine positive Ladung am Stickstoffatom oder im aromatischen Ringsystem notwendig ist, sind die Stickstoffatome jeweils durch ein Wasserstoffatom oder eine organische Gruppen mit im Allgemeinen nicht mehr als 20 C-Atomen, vorzugsweise eine Kohlenwasserstoffgruppe, insbesondere eine C1 bis C16 Alkylgruppe, insbesondere eine C1 bis C10, besonders bevorzugt eine C1 bis C4 Alkylgruppen substituiert.

Auch die Kohlenstoffatome des Ringsystems können durch organische Gruppen mit im Allgemeinen nicht mehr als 20 C-Atomen, vorzugsweise eine Kohlenwasserstoffgruppe, insbesondere eine C1 bis C16 Alkylgruppe, insbesondere eine C1 bis C10, besonders bevorzugt eine C1 bis C4 Alkylgruppen substituiert sein.

Besonders bevorzugt handelt es sich bei dem organischen Kation um Imidazolium-Kationen der nachstehenden Formel I handelt, 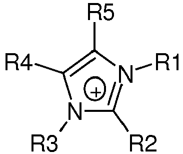 worin
R1 für einen organischen Rest mit 1 bis 20 C-Atomen steht und
R2, R3, R4 und R5 für ein H-Atom oder einen organischen Rest mit 1 bis 20 C-Atomen stehen.

In Formel I stehen R1 und R3 vorzugsweise unabhängig für einen organischen Rest mit 1 bis 10 C-Atomen. Insbesondere stehen R1 und R3 für einen aliphatischen Rest, insbesondere einen aliphatischen Rest ohne weitere Heteroatome, z.B. für eine Alkylgruppe. Besonders bevorzugt stehen R1 und R3 unabhängig voneinander für eine C1- bis C10- bzw. eine C1 bis C4 Alkylgruppe. In einer besonderen Ausführungsform sind R1 und R3 identisch und stehen insbesondere für identische C1- bis C10- bzw. C1- bis C4- Alkylgruppen.

In Formel I stehen R2, R4 und R5 vorzugsweise unabhängig für ein H-Atom oder einen organischen Rest mit 1 bis 10 C-Atomen; insbesondere stehen R2, R4 und R5 für ein H-Atom oder einen aliphatischen Rest. Besonders bevorzugt stehen R2, R4 und R5 unabhängig voneinander für ein H-Atom oder eine Alkylgruppe, insbesondere stehen R2, R4 und R5 unabhängig voneinander für ein H-Atom oder eine C1 bis C4 Alkylgruppe. Ganz besonders bevorzugt stehen R2, R4 und R5 jeweils für ein H-Atom.

### Zum Anion Y⁻

Das Ausgangssalz K⁺ Y⁻ enthält das organische Anion Y⁻.

Y⁻ steht für ein organisches Anion mit einer Carboxylat-, Sulfonat- oder Sulfatgruppe.

Das Anion Y⁻ enthält insgesamt mindestens 4, besonders bevorzugt mindestens 6 C-Atome. Im Allgemeinen enthält Y⁻ nicht mehr als 30, insbesondere nicht mehr als 20 C-Atome. In einer bevorzugten Ausführungsform enthält Y⁻ außer den Sauerstoffatomen und Schwefelatomen in der Carboxylat-, Sulfonat- oder Sulfatgruppe keine weiteren Heteroatome. Insbesondere besteht das Anion Y⁻ aus einer anionischen Gruppe ausgewählt aus einer Carboxylat-, einer Sulfonat- oder einer Sulfatgruppe und darüber hinaus aus einem Kohlenwasserstoffrest ohne weitere Heteroatome oder funktionelle Gruppen.

Geeignete Anionen mit einer Sulfatgruppe sind insbesondere Anionen der Formel

Rₐ-O-SO₃⁻, wobei Rₐ für eine C4 bis C20- Alkylgruppe, besonders bevorzugt für eine C6- bis C20-Alkylgruppe steht.

Geeignete Anionen mit einer Sulfonatgruppe sind insbesondere Anionen der Formel

R_{b}-SO₃⁻, wobei R_{b} für eine C4 bis C20- Alkylgruppe, besonders bevorzugt für eine C6 bis C20-Alkylgruppe steht.

Vorzugsweise handelt es sich bei dem Anion Y⁻ um ein Anion mit einer Carboxylatgruppe.

Es kann sich dabei sowohl um aliphatische als auch um aromatische Carboxylate handeln, wobei unter den aromatischen Carboxylate solche verstanden werden, die aromatische Gruppen enthalten. Besonders bevorzugt sind aliphatische oder aromatische Carboxylate, die außer den Sauerstoffatomen der Carboxylatgruppe keine weiteren Heteroatome enthalten oder allenfalls noch ein oder zwei Hydroxylgruppen, Carbonylgruppen oder Ethergrupen enthalten. Als letztere genannt seien z.B. Hydroxycarboxylate oder Ketocarboxylate.

Als Carboxylate mit derartigen weiteren Heteroatomen seien z.B. die Carboxylate der Glycolsäure, Furandicarbonsäure, Levulinsäure (4-Oxopentansäure) genannt.

Besonders bevorzugt sind aliphatische oder aromatische Carboxylate, die außer den Sauerstoffatomen der Carboxylatgruppe keine weiteren Heteroatome enthalten, z.B. die Carboxylate der Alkancarbonsäuren, Alkencarbonsäuren, Alkincarbonsäuren, Alkadiencarbonsäuren, Alkatriencarbonsäuren, , Benzoesäure oder Phenylessigsäure. Geeignete Carboxylate der Alkancarbonsäuren, Alkencarbonsäuren und Alkadiencarbonsäuren sind auch als Fettsäure- Carboxylate bekannt.

Genannt seien insbesondere die Carboxylate n-Buttersäure (C4-Carbonsäure), n-Valeriansäure (C5-Carbonsäure), n-Capronsäure (C6-Carbonsäure) n-Caprylsäure (C8-Carbonsäure, Octansäure), n-Caprinsäure (C10-Carbonsäure, Decansäure), Laurinsäure (C12-Carbonsäure, Dodecansäure), Palmitinsäure (C16-Carbonsäure, Hexadecansäure) oder Stearinsäure (C18-Carbonsäure). In einer besonderen Ausführungsform handelt es sich bei den Anionen der Salze um Carboxylate der C6 bis C20 Alkancarbonsäuren, d.h. C6 - C20 Alkanoate. Genannt seien insbesondere C6 bis C14 Alkanoate und in einer besonderen Ausführungsform C8 bis C12 Alkanoate.

Als Ausgangssalze K⁺ Y⁻ seien exemplarisch genannt:
1-Ethyl-3-methyl-imidazolium octanoat,
   1-Methyl-3-methyl-imidazolium octanoat,
   1-Ethyl-3-ethyl-imidazolium octanoat,
1-Ethyl-3-methyl-imidazolium ethylhexanoat,
   1-Methyl-3-methyl-imidazolium ethylhexanoat,
   1-Ethyl-3-ethyl-imidazolium ethylhexanoat,
1-Ethyl-3-methyl-imidazolium isononanoat,
   1-Methyl-3-methyl-imidazolium isononanoat,
   1-Ethyl-3-ethyl-imidazolium isononanoat,

Besonders bevorzugt sind Imidazoliumsalze mit einem Imidazoliumkation, in dem R1 und R3 identisch sind (siehe oben) und einem C6- bis C20-Alkanoat (bzw. C6 bis C14 oder C8 - C12 Alkanoat) als Anion.

### Zur Säure HX

Das Ausgangssalz K⁺ Y⁻ wird mit einer Wasserstoffsäure HX umgesetzt wird, deren pKs - Wert kleiner ist als der pKs Wert der Wasserstoffsäure HY. HY ist dabei die zu den obigen Anionen Y⁻ gehörende Wasserstoffsäure.

Säuren HX sind ausgewählt aus den Säuren:

HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄.

In einer bevorzugten Ausführungsform ist die Wasserlöslichkeit von HX größer als die Wasserlöslichkeit von HY.

Die Umsetzung kann z.B. bei Raumtemperatur oder auch erhöhter Temperatur (z.B. bei ionischen Flüssigkeiten mit einem Schmelzpunkt oberhalb Raumtemperatur) erfolgen. Vorzugsweise erfolgt eine Umsetzung bei Normalbedingungen, insbesondere bei 15 bis 30°C und Normaldruck.

Die Säure HX wird zum Ausgangssalz K⁺ Y⁻ gegeben. Sowohl HX als auch Ausgangssalz K⁺ Y⁻ können im Überschuss eingesetzt werden. Das Molverhältnis von HX zu Ausgangssalz K⁺ Y⁻ kann z.B. 0, 1 bis 1 bis 1 zu 0,1 betragen. Da die Umsetzung equimolar ist, ist ein Molverhältnis von 0,8 zu 1 bis 1 zu 0,8 bevorzugt, um einen zu großen Überschuss einer nicht umgesetzten Verbindung zu vermeiden.

Bei der Durchführung können Lösemittel mitverwendet werden.

Vorzugsweise erfolgt die Umsetzung in Gegenwart eines Lösemittels, in dem das gebildete Salz K⁺ X⁻löslich ist. Insbesondere handelt es sich dabei um Wasser oder ein mit Wasser zumindest zum Teil homogen mischbares, hydrophiles organisches Lösemittel oder deren Gemische (nachfolgend zusammenfassend wässriges Lösemittel genannt).

Als derartige hydrophile Lösemittel seien z.B. aliphatische Alkohole oder Ether mit maximal 4 Kohlenstoffatomen, z.B. Methanol, Ethanol, Methylethylether oder Tetrahydrofuran genannt. Geeignete hydrophile Lösemittel haben eine Löslichkeit in Wasser von mindestens 50 Gramm(g), vorzugsweise von mindestens 100 g, insbesondere von mindestens 200 g in ein Liter Wasser (bei 21°C, 1 bar). In einer bevorzugten Ausführungsform ist das hydrophile Lösemittel mit Wasser in jedem Verhältnis mischbar (21°C, 1 bar).

Als wässriges Lösemittel bevorzugt ist Wasser.

Bei der Umsetzung kann zusätzlich auch ein hydrophobes organisches Lösemittel mitverwendet werden, in dem das gebildete HY löslich ist. Dabei handelt es sich insbesondere um organische, mit Wasser nicht oder wenig mischbare Lösemittel Derartige geeignete hydrophobe Lösemittel haben eine Löslichkeit in Wasser von weniger als 200 g, insbesondere weniger als 100 g, besonders bevorzugt weniger als 50 g pro Liter Wasser (21°C, 1 bar). Erwähnt seien z.B. Methyltertiärbutylether (MTBE) und insbesondere Kohlenwasserstoffe, wie Alkane, z.B. Heptan, Gemische von Kohlenwasserstoffen, z.B. Benzine.

Das wässrige und hydrophobe Lösemittel sind nicht oder nur wenig mischbar. Sie bilden zwei getrennte flüssige Phasen aus.

In einer bevorzugten Ausführungsform erfolgt die Umsetzung in Gegenwart eines wässrigen Lösemittels. Nach der Umsetzung fallen dann zwei flüssige Phasen an: die Lösung von K⁺ X⁻ in dem wässrigen Lösemittel und als davon getrennte organische Phase die Säure HY.

In einer ebenso bevorzugten Ausführungsform erfolgt die Umsetzung in Gegenwart sowohl eines wässrigen und als auch eines hydrophoben Lösemittels. Nach der Umsetzung fallen dann wiederum zwei separate flüssige Phasen an: die Lösung von K⁺ X⁻ in dem wässrigen Lösemittel und als davon getrennte organische Phase die Lösung von HY in dem hydrophoben Lösemittel.

Bevorzugt ist ein Verfahren, bei dem ein Ausgangssalz K⁺ Y⁻ mit den oben als bevorzugt genannten Kationen K⁺ und/oder den oben bevorzugt genannten Anionen Y⁻, besonders bevorzugt ein Imidazolium- C6- bis C20-alkanoat mit einer Säure HX umgesetzt wird, wobei es sich bei HX um die oben aufgeführten Säuren handelt.

Gesamtverfahren zur Herstellung von Imidazoliumsalzen

Das erfindungsgemäße Verfahren eignet sich, wie vorstehend ausgeführt, zur Herstellung von Imidazoliumsalzen mit unterschiedlichem Anion.

Das erfindungsgemäße Verfahren kann sich insbesondere als weiterer Verfahrensschritt an die Herstellung von Imidazoliumsalzen anschließen.

Die Herstellung von Imidazoliumsalzen kann in einem Verfahrensschritt durchgeführt werden, wie aus WO 91/14678 und WO 2009/074535 bekannt ist.

Salze K⁺ X⁻, wobei K⁺ ein Imidazolium-Kation der Formel I, worin R1 und R3 unanhängig voneinander für einen organischen Rest stehen (R3 also nicht H bedeutet), bezeichnet und X- ein Anion bezeichnet, können daher durch ein Verfahren hergestellt werden, welches dadurch gekennzeichnet, dass
a) zunächst ein Imidazoliumsalz K⁺ Y⁻ wobei Y⁻ für ein organisches Anion mit einer Carboxylat-, Sulfonat- oder Sulfatgruppe steht und das Anion Y⁻ insgesamt mindestens 4 C-Atome enthält, durch Umsetzung einer α -Dicarbonylverbindung, eines Aldehyd, eines Amins und der Wasserstoffsäure HY hergestellt wird und HX ausgewählt ist aus HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄ und
b) das so erhaltene Imidazoliumsalz K⁺ Y⁻ mit einer Wasserstoffsäure HX umgesetzt wird, deren pKs - Wert kleiner ist als der pKs Wert der Wasserstoffsäure HY, und
   - nach der Umsetzung das erhaltene Salz K⁺ X⁻ und die erhaltene Wasserstoffsäure HY in getrennten flüssigen Phasen vorliegen.

Die Variablen K⁺ Y⁻, K⁺ X⁻, HX und HY haben dabei die obigen Bedeutungen und bevorzugten Bedeutungen.

Besonders gut können nach diesem Verfahren in Stufe a) Imidazoliumcarboxylate K⁺ Y⁻ hergestellt werden, in denen Y⁻ für Anionen mit einer Carboxylatgruppe steht. Für bevorzugte Anionen mit einer Carboxylatgruppe gelten die obigen Ausführungen.

In Stufe a) werden die Ausgangsverbindungen entsprechend den gewünschten Resten R1 bis R5 in Formel I gewählt.

Bei der α -Dicarbonylverbindung handelt es sich vorzugsweise um eine Verbindung der Formel II

R4-CO-CO-R5 ,

wobei R4 und R5 die oben genannte Bedeutung haben.

Besonders bevorzugt handelt es sich um Glyoxal.

Bei dem Aldehyd handelt es sich insbesondere um einen Aldehyd der Formel R2-CHO, wobei R2 die oben genannte Bedeutung hat. Besonders bevorzugt ist Formaldehyd; der Formaldehyd kann auch in Form von Formaldehyd freisetzenden Verbindungen wie Paraformaldehyd oder Trioxan eingesetzt werden.

Bei den Aminen handelt es sich insbesondere um primäre Amine des Typs R- NH2. Der Rest R entspricht den Resten R1 und R3 der erhaltenen Imidazoliumsalze. Wenn ein definiertes primäres Amin eingesetzt wird, erhält man ein Imidazoliumsalz, in dem die Reste R1 und R3 identisch sind. Verwendet man ein Gemisch von Aminen (z.B. Gemisch aus R'-NH2 und R"-NH2) so erhält man ein Gemisch von Imidazoliumsalzen (Gemisch von Salzen mit R1 und R3 = R', R1 und R3 = R" und Salzen mit R1 = R' und R3 = R").

Bei der Wasserstoffsäure handelt es sich um die Wasserstoffsäure des Anions Y⁻, vorzugsweise um eine Alkancarbonsäure, insbesondere um eine C6- bis 20- Alkancarbonsäure, z.B. eine C6 bis C14- Alkancarbonsäure.

### Zur Durchführung des Verfahrens

Die Umsetzung der Ausgangsverbindungen kann in einem geeigneten Lösemittel, vorzugweise in Wasser, einem mit Wasser mischbaren Lösemittel oder deren Gemische erfolgen.

Als mit Wasser mischbare Lösemittel seien insbesondere protische Lösemittel, vorzugsweise aliphatische Alkohole oder Ether mit maximal 4 Kohlenstoffatomen, z.B. Methanol, Ethanol, Methylethylether, Tetrahydrofuran genannt. Geeignete protische Lösemittel sind mit Wasser in jedem Verhältnis mischbar (bei 1 bar, 21°C).

Vorzugsweise erfolgt die Umsetzung in Wasser oder Gemischen von Wasser mit den vorstehenden protischen Lösemitteln. Besonders bevorzugt erfolgt die Umsetzung in Wasser.

Die Umsetzung der Ausgangskomponenten kann bei Normaldruck und z.B. bei Temperaturen von 5 bis 100°C, insbesondere 5 bis 50°C, besonders bevorzugt 10 bis 40°C erfolgen.

Aus der erhaltenen Produktmischung können die Imidazoliumsalze K⁺ Y⁻ abgetrennt werden. Wenn es sich bei dem Anion Y⁻ um eine Verbindung mit einer Carboxylatgruppe handelt, kann dies sehr einfach durch eine Molekulardestillation, wie sie in WO 2009/027250 für Imidazoliumcarboxylate beschrieben ist, erfolgen.

Daran schließt sich dann in Verfahrensschritt b) der gewünschte Anionenaustausch an. Dieser kann durchgeführt werden wie oben beschrieben ist.

Das erfindungsgemäße Verfahren ist ein einfaches Verfahren zur Herstellung von ionischen Flüssigkeiten, insbesondere Imidazoliumsalzen mit unterschiedlichem Anion. Insbesondere können Imidazoliumsalze K⁺X⁻ in nur zwei Stufen durch Umsetzung der Ausgangsverbindungen (α -Dicarbonylverbindung, Aldehyd, Amin, HY) zu K⁺ Y⁻ und einen anschließenden Anionentausch mit einer Säure HX hergestellt werden.

### Beispiele

### Beispiel 1

### Herstellung von 1-Ethyl-3-Ethyl-Imidazolium - Chlorid (1,3-EEIM-Chlorid)

### Reaktionsgleichung:

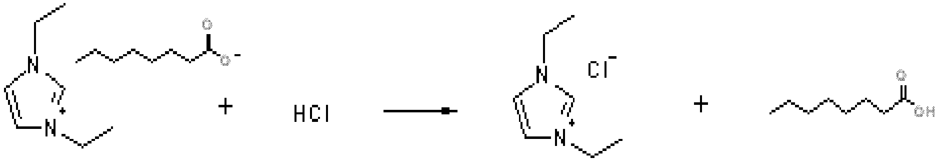

### Molgewichte

1,3-EEIM-Octanoat: 268,39g/mol
HCl: 36,45g/mol
1,3-EEIM-Chlorid: 160,61g/mol
Octansäure: 144,24g/mol
Rührgeschwindigkeiten: 250 U/min
Apparatur: 5l Scheidetrichter, Halbmondrührer

**Ansatz:**

| | | | | Substanz | |
|---|---|---|---|---|---|
| 2784 | g | 10,37 | mol | 1,3-Diethyl-imidazolium-octanoat | |
| 1000 | g | 55,52 | mol | Wasser | |
| 1058 | g | 10,74 | mol | Salzsäure 37%ig | 1,04eq bez. auf EEIM-Octanoat |

### Durchführung:

1,3-EEIM-Octanoat und Wasser wurden im Scheidetrichter vorgelegt. Unter Rühren wurde die Salzsäure hinzugegeben. Es entstand ein weißer Nebel und das Gemisch wurde heiß. Es wurde noch 10 min weitergerührt, bis sich die zwei entstandenen Phasen sauber getrennt hatten.

Die obere Phase war die organische Phase, sie bestand aus Octansäure.

Die unter Phase war die wässrige Phase, sie bestand aus der wässrigen Lösung des entstandenen Imidazoliumchlorids.

Die untere Phase enthielt nur noch 0,55 Gew. % Octanoat (gaschromatographisch bestimmt).

### Beispiel 2

### Herstellung von 1,3-EEIM-Formiat

### Reaktionsgleichung:

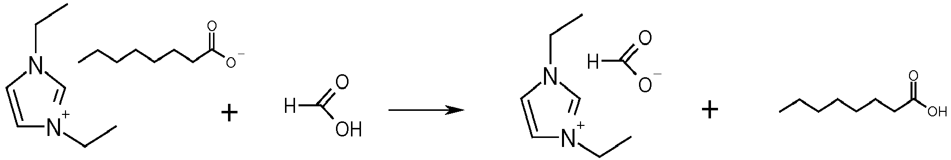

### Molgewichte

1,3-EEIM-Octanoat: 268,39 g/mol
Ameisensäure: 46,03 g/mol
1,3-EEIM-Formiat: 170,18g/mol
Octansäure: 144,24g/mol
Apparatur: 51 Scheidetrichter

**Ansatz:**

| | | Substanz |
|---|---|---|
| 936,7 g | 3,49 mol | 1,3-Diethyl-imidazolium-octanoat |
| 1700 g | | Wasser |
| 1000 ml | | MTBE |
| 161,0 g | 3,50 mol | Entspr. 189,4 g Ameisensäure 85%ig |

### Durchführung:

Methyltertiärbutylether (MTBE), Wasser, EEIM-Octanoat und Ameisensäure wurden in einen 51 Scheidetrichter gegeben und ca. 5min geschüttelt. Danach wurde die erhaltene Emulsion ca. 10 min stehen gelassen. Es entstanden 2 scharf getrennte Phasen.

Die obere Phase war die organische Phase, sie bestand aus der Lösung von Octansäure in MTBE.

Die untere wässrige Phase wurde abgetrennt. In dieser Phase befand sich das gebildete EEIM-Formiat. In der unteren Phase konnte gaschromatographisch kein Octanoat mehr nachgewiesen. (Nachweisgrenze 0,01 %).

Die Masse der Unterphase betrug 2312,5g (Theorie: 595,74g EEIM-Formiat + 1728,4g H2O =2324,2g).

## Patentansprüche

1. Verfahren zur Herstellung von Salzen K⁺ X-, wobei es sich bei K⁺ um ein organisches Kation mit einem heterocyclischen Ringsystem und mindestens einem Stickstoffatom als Bestandteil des Ringsystems handelt und X- ein Anion bezeichnet, durch Anionenaustausch, **dadurch gekennzeichnet, dass**
- von einem Salz K⁺ Y⁻ ausgegangen wird, wobei K⁺ die obige Bedeutung hat und Y⁻ für ein organisches Anion mit einer Carboxylat-, Sulfonat- oder Sulfatgruppe steht, das Anion Y⁻ insgesamt mindestens 4 C-Atome enthält und
- K⁺ Y⁻ mit einer Wasserstoffsäure HX umgesetzt wird, deren pKs - Wert kleiner ist als der pKs Wert der Wasserstoffsäure HY, und HX ausgewählt ist aus HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄.
- nach der Umsetzung das erhaltene Salz K⁺ X⁻ und die erhaltene Wasserstoffsäure HY in getrennten flüssigen Phasen vorliegen.

2. Verfahren gemäß einem der Ansprüche 1, **dadurch gekennzeichnet, dass** es sich bei K⁺ um ein Imidazolium-kation der nachstehenden Formel I handelt, 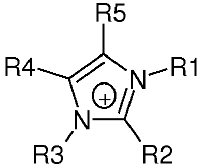 worin
R1 für einen organischen Rest mit 1 bis 20 C-Atomen steht und
R2, R3, R4 und R5 für ein H-Atom oder einen organischen Rest mit 1 bis 20 C-Atomen stehen.

3. Verfahren gemäß einem der Ansprüche 1 oder 2 , **dadurch gekennzeichnet, dass** es sich bei K⁺ um ein Imidazolium-Kation der Formel I handelt, wobei R1 und R3 identisch sind und für eine C1 - bis C10-Alkylgruppe stehen.

4. Verfahren gemäß einem der Ansprüche 1 bis 3, **dadurch gekennzeichnet, dass** es sich bei Y⁻ um ein C6- bis C20- Alkanoat handelt.

5. Verfahren gemäß einem der Ansprüche 1 bis 4, **dadurch gekennzeichnet, dass** die Wasserlöslichkeit von HX größer ist als die Wasserlöslichkeit von HY.

6. Verfahren gemäß einem der Ansprüche 1 bis 5, **dadurch gekennzeichnet, dass** die Umsetzung in Gegenwart eines Lösemittels erfolgt, in dem das gebildete Salz K⁺ X⁻löslich ist.

7. Verfahren gemäß Anspruch 6, **dadurch gekennzeichnet, dass** es sich bei dem Lösemittel um Wasser handelt.

8. Verfahren gemäß einem der Ansprüche 1 bis 7, **dadurch gekennzeichnet, dass** die Umsetzung in Gegenwart eines organischen Lösemittels erfolgt, in dem das gebildete HY löslich ist.

9. Verfahren gemäß einem der Ansprüche 1 bis 8, **dadurch gekennzeichnet, dass** die Umsetzung in Gegenwart eines Lösemittels erfolgt, in dem das gebildete Salz K⁺ X⁻löslich ist und in Gegenwart eines organischen Lösemittels erfolgt, in dem das gebildete HY löslich ist.

10. Verfahren gemäß Anspruch 1, **dadurch gekennzeichnet, dass** K⁺ ein 1,3 disubstituiertes Imidazolium-Kation und X- ein Anion bezeichnet und
a) zunächst ein Imidazoliumsalz K⁺ Y⁻, wobei Y⁻ für ein organisches Anion mit einer Carboxylat-, Sulfonat- oder Sulfatgruppe steht und das Anion Y⁻ insgesamt mindestens 4 C-Atome enthält, durch Umsetzung einer α -Dicarbonylverbindung, eines Aldehyd, eines Amins und der Wasserstoffsäure HY hergestellt wird und
b) das so erhaltene Imidazoliumsalz K⁺ Y⁻ mit einer Wasserstoffsäure HX umgesetzt wird, deren pKs - Wert kleiner ist als der pKs Wert der Wasserstoffsäure HY und HX ausgewählt ist aus HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄.
und
- nach der Umsetzung das erhaltene Salz K⁺ X⁻ und die erhaltene Wasserstoffsäure HY in getrennten flüssigen Phasen vorliegen.

## Claims

1. A process for preparing salts K⁺ X⁻, where K⁺ is an organic cation having a heterocyclic ring system and at least one nitrogen atom as constituent of the ring system and X⁻ is an anion, by anion exchange, wherein
- a salt K⁺ Y⁻, where K⁺ is as defined above and Y⁻ is an organic anion having a carboxylate, sulfonate or sulfate group, the anion Y⁻ comprises a total of at least 4 carbon atoms, is used as starting material and
- K⁺ Y⁻ is reacted with a hydrogen acid HX whose pKₐ is less than the pKₐ of the hydrogen acid HY and HX is selected from among HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄,
- after the reaction, the salt K⁺ X⁻ obtained and the hydrogen acid HY obtained are present in separate liquid phases.

2. The process according to one of claims 1, wherein K⁺ is an imidazolium cation of the formula I, 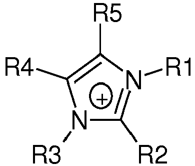 where
R1 is an organic radical having from 1 to 20 carbon atoms and
R2, R3, R4 and R5 are each an H atom or an organic radical having from 1 to 20 carbon atoms.

3. The process according to either of claims 1 and 2, wherein K⁺ is an imidazolium cation of the formula I in which R1 and R3 are identical and are each a C1-C10-alkyl group.

4. The process according to any of claims 1 to 3, wherein Y⁻ is a C6-C20-alkanoate.

5. The process according to any of claims 1 to 4, wherein the solubility of HX in water is greater than the solubility of HY in water.

6. The process according to any of claims 1 to 5, wherein the reaction is carried out in the presence of a solvent in which the salt K⁺ X⁻ formed is soluble.

7. The process according to claim 6, wherein the solvent is water.

8. The process according to any of claims 1 to 7, wherein the reaction is carried out in the presence of an organic solvent in which the HY formed is soluble.

9. The process according to any of claims 1 to 8, wherein the reaction is carried out in the presence of a solvent in which the salt K⁺ X⁻ formed is soluble and in the presence of an organic solvent in which the HY formed is soluble.

10. The process according to claim 1, wherein K⁺ is a 1,3-disubstituted imidazolium cation and X⁻ is an anion and
a) an imidazolium salt K⁺ Y⁻, where Y⁻ is an organic anion having a carboxylate, sulfonate or sulfate group and the anion Y⁻ comprises a total of at least 4 carbon atoms, is firstly prepared by reaction of an α-dicarbonyl compound, an aldehyde, an amine and the hydrogen acid HY and
b) the imidazolium salt K⁺ Y⁻ obtained in this way is reacted with a hydrogen acid HX whose pKₐ is less than the pKₐ of the hydrogen acid HY and HX is selected from among HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO_{4,} and
- after the reaction, the salt K⁺ X⁻ obtained and the hydrogen acid HY obtained are present in separate liquid phases.

## Revendications

1. Procédé pour la préparation de sels K⁺X⁻, où il s'agit, pour K⁺, d'un cation organique présentant un système cyclique hétérocyclique et au moins un atome d'azote comme constituant du système cyclique, et X⁻ désigne un anion, par échange d'anions, **caractérisé en ce que**
- on part d'un sel K⁺Y⁻, K⁺ présentant la signification ci-dessus et Y⁻ représentant un anion organique présentant un groupe carboxylate, sulfonate ou sulfate, l'anion Y⁻ contenant au total au moins 4 atomes de carbone et
- on transforme K⁺Y⁻ avec un hydracide HX dont la valeur pKa est inférieure à la valeur pKa de l'hydracide HY et HX étant choisi parmi HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄,
- après la transformation, le sel obtenu K⁺X⁻ et l'hydracide obtenu HY se trouvent dans des phases liquides séparées.

2. Procédé selon l'une des revendications 1, **caractérisé en ce qu'**il s'agit, pour K⁺, d'un cation d'imidazolium de la formule I suivante, 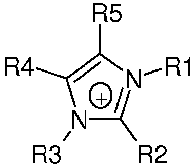 dans laquelle
R1 représente un radical organique comprenant 1 à 20 atomes de carbone et
R2, R3, R4 et R5 représentent un atome d'hydrogène ou un radical organique comprenant 1 à 20 atomes de carbone.

3. Procédé selon l'une quelconque des revendications 1 ou 2, **caractérisé en ce qu'**il s'agit, pour K⁺, d'un cation imidazolium de formule I, R1 et R3 étant identiques et représentant un groupe C₁-C₁₀-alkyle.

4. Procédé selon l'une quelconque des revendications 1 à 3, **caractérisé en ce qu'**il s'agit, pour Y⁻, d'un alcanoate en C₆-C₂₀.

5. Procédé selon l'une quelconque des revendications 1 à 4, **caractérisé en ce que** la solubilité dans l'eau de HX est supérieure à la solubilité dans l'eau de HY.

6. Procédé selon l'une quelconque des revendications 1 à 5, **caractérisé en ce que** la transformation a lieu en présence d'un solvant dans lequel le sel formé K⁺X⁻ est soluble.

7. Procédé selon la revendication 6, **caractérisé en ce qu'**il s'agit, pour le solvant, d'eau.

8. Procédé selon l'une quelconque des revendications 1 à 7, **caractérisé en ce que** la transformation a lieu en présence d'un solvant organique dans lequel l'hydracide formé HY est soluble.

9. Procédé selon l'une quelconque des revendications 1 à 8, **caractérisé en ce que** la transformation a lieu en présence d'un solvant dans lequel le sel formé K⁺X⁻ est soluble et en présence d'un solvant organique dans lequel l'hydracide formé HY est soluble.

10. Procédé selon la revendication 1, **caractérisé en ce que** K⁺ désigne un cation d'imidazolium disubstitué en positions 1,3 et X⁻ désigne un anion et
a) on prépare d'abord un sel d' imidazolium K⁺Y⁻, Y⁻ représentant un anion organique présentant un groupe carboxylate, sulfonate ou sulfate et l'anion Y⁻ contenant au total au moins 4 atomes de carbone, par transformation d'un composé α-dicarbonyle, d'un aldéhyde, d'une amine et de l'hydracide HY et
b) on transforme le sel d'imidazolium ainsi obtenu K⁺Y⁻ avec un hydracide HX dont la valeur pKa est inférieure à la valeur pKa de l'hydracide HY et HX étant choisi parmi HCl, HBr, HBF₄, H₃C-COOH, HCOOH, H₃C-O-SO₃H, H₃C-SO₃H, F₃C-O-SO₃H, HPF₆, CH₃-CH₂-COOH, HSCN, H₂SO₃, HNO₃, HClO₄,
et
- après la transformation, le sel obtenu K⁺X⁻ et l'hydracide formé HY se trouvent dans des phases liquides séparées.
